# EUROPÄISCHE PATENTSCHRIFT

(11) **EP 1 663 347 B1**
(45) Veröffentlichungstag und Bekanntmachung des Hinweises auf die Patenterteilung: **02.01.2008**
(21) Anmeldenummer: 04761033.2
(22) Anmeldetag: 13.09.2004
(51) Int. Cl.: A61M 1/36, B01D 15/08

(54) **APHERESE-VORRICHTUNG**
APHERESIS DEVICE
DISPOSITIF DE PRÉLÈVEMENT PAR APHÉRÈSE

(30) Priorität: 12.09.2003 AT 14442003
(43) Veröffentlichungstag der Anmeldung: 07.06.2006
(73) Patentinhaber: AFFIRIS Forschungs- und Entwicklungs GmbH, 1030 Wien (AT)
(72) Erfinder: MATTNER, Frank, 1190 Wien (AT); SCHMIDT, Walter, 1030 Wien (AT)
(74) Vertreter: Sonn & Partner Patentanwälte
(86) Internationale Anmeldenummer: PCT/AT2004/000311
(87) Internationale Veröffentlichungsnummer: WO 2005/025651

(56) Entgegenhaltungen:
- WO-A-03/051374
- WO-A-20/04056318
- US-A- 4 409 105
- US-A- 4 770 774
- US-A- 5 216 127

## Beschreibung

Die Erfindung betrifft eine Apherese-Vorrichtung, umfassend einen mit dem Blut- oder Plasmafluss kontaktierbaren festen Träger.

Unter Apherese werden Behandlungsverfahren verstanden, deren Therapieeffekte auf der extrakorporalen Elimination pathogener Proteine, proteingebundener pathogener Substanzen, freier pathogener Substanzen oder pathogener Zellen des Blutes beruhen. Wenn das pathogene Protein nur aus dem zellfreien Plasma eliminiert werden kann, wird das Plasma vorher von den Blutzellen mit Hilfe eines Membranplasmaseparators (Plasmaseparation) oder mit Hilfe einer Hämozentrifuge getrennt. Beim unselektiven Plasmaaustausch (Plasmapherese) wird das ausgetauschte Patientenplasma als gesamtes separiert, wobei neben den Pathogenen auch alle anderen lebensnotwendigen Proteine eliminiert werden. Dadurch ist die Substituierung des entnommenen Plasmas mit Elektrolyten, Humanalbumin oder Frischplasma erforderlich. Bei selektiven Plasmaphereseverfahren können mit Hilfe von Adsorption, Präzipitation oder Filtration pathogene Proteine ganz spezifisch aus dem separierten Plasma entfernt werden, wobei das Plasma nach der erfolgten Entfernung ohne wesentlichen Volumenverlust reinfundiert werden kann. Diese selektiven Verfahren haben den Vorteil, dass hierbei auf eine Substitutionslösung verzichtet werden kann. Bei selektiven Vollblutaphereseverfahren werden die pathogenen Eiweiße spezifisch und ohne vorherige Plasmaseparation direkt aus dem nicht vorbehandelten Blut adsorbiert, womit - im Gegensatz zu den Plasmaseparationsverfahren - sowohl die Plasmaseparation als auch die Zugabe einer Substitutionslösung entfallen können. Eine weitere Unterform der Apherese ist die Zytapherese, bei welcher Zellen aus dem Blut entfernt werden. Dabei können selektiv Leukozyten, Erythrozyten, Thrombozyten, Granulozyten oder sogar Stammzellen gewonnen werden.

Obgleich die Apherese (z.B. als Plasmapherese oder Zytapherese) gegenwärtig vorwiegend zur Gewinnung von Spenderplasma (als Plasmakonserve, zur Isolierung verschiedener Plasmafraktionen oder zur Gewinnung von Blutprodukten) verwendet wird, gewinnen Aphereseverfahren zunehmend auch auf therapeutischem Gebiet an Bedeutung. So werden gegenwärtig eine ganze Reihe von Stoffwechselkrankheiten (z.B. (familiäre) Hypercholesterinämie, progrediente koronare Herzkrankheit mit isolierter Lp(a)-Erhöhung, Chylomikronämie-Syndrom, Leberversagen,...), Nierenkrankheiten (Goodpasture Syndrom, Systemischer Lupus erythematodes mit Lupusnephritis, Wegnersche Granulomatose, Hämolytisch-urämisches Syndrom, Idiopathische fokal-sklerosierende Glomerulonephritis, Paraproteinämie assoziierte Syndrome, Kryoglobulinämische Purpura, HLA-Sensibilisierung bei Nierentransplantation,...), Erkrankungen des Nervensystems (Myasthenia gravis, Guillain-Barri-Syndrom, Chronische demyelinisierende Polyradikuloneuritis, Paraproteinämische Polyneuropathie, Lambert-Eaton-Syndrom, Refsum-Syndrom,...), Erkrankungen des Immunsystems (Rheumatoide Arthritis, Hemmkörperhämophilie, Pemphigus,...), Erkrankungen des Blutkreislaufs und der Mikrozirkulation (Hyperviskositätssyndrom, Antiphospholipidantikörper-Syndrom, Thrombotische Mikroangiopathie nach Knochenmarktransplantation, Altersabhängige Makuladegeneration, Hörsturz, Periphere Störungen der Mikrozirkulation, Idiopathische dilatative Kardiomyopathie, Transplantatvaskulopathie nach Herztranspantation, Homozygote familiäre Hypercholesterinämie, Fokal segmentale Glomerulosklerose, Hämolytisch-urämisches Syndrom,...), Vergiftungen, akute Leberinsuffizienz, Neoplasmen, Hyperhydratation, Thyreotoxikose, etc., mit Aphereseverfahren behandelt (siehe Pschyrembel (257. Auflage) Stichwort "Plasmapherese"; www.nephrologie.de/172Apharese.htm).

Die Alzheimer'sche Erkrankung (AE) ist eine progressive, neurologische Störung für die gegenwärtig keine effektive Behandlung möglich ist. Typisch für diese Erkrankung sind zerebrale Plaques, welche das Amyloid-β-Peptid enthalten, und fadenartige neuronale Strukturen aus dem Mikrotubulus assoziierten TAU-Protein. Obgleich sowohl Amyloid-β und TAU für die Pathogenese als relevant betrachtet werden, scheinen die aktuellsten Forschungsergebnisse darauf hinzudeuten, dass Amyloid-β das vorrangige Agens in der Pathogenese darstellt. Es werden daher zunehmend Therapeutika entwickelt, die die Amyloid-β-Produktion, die Amyloid-β Aggregation oder die von diesen Aggregaten verursachten neurotoxischen Vorkommnisse verhindern sollen. Eine zusammenfassende Darstellung der bislang eingeschlagenen therapeutischen Strategien für AE ist im Übersichtsartikel von Wolfe (Nature Reviews Drug Discovery 1 (2002) 859-866) gegeben.

Die Amyloid-β-Plaques bilden sich ausgehend vom so genannten Amyloid-β Precurser Protein (APP), welches ein integrales Transmembran-Protein ist (für das auch keine physiologische Funktion klar belegt ist; allerdings lassen neueste Forschungsergebnisse vermuten, dass APP als sog. Membran-Cargo-Rezeptor für Kinesin I fungiert). APP wird durch so genannte Sekretasen proteolytisch gespalten, wobei physiologisch vor allem ein 40-Aminosäuren langes Aß-Peptid (Aβ₄₀) gebildet wird. Andere, kürzere und längere Formen von Aβ entstehen ebenfalls, vor allem eine 42-Aminosäure-Version (Aβ₄₂), welche ein hohes Aggregationsvermögen aufweist. Diese Aβ₄₂-Form ist daher auch die überwiegende Form in amyloiden Plaques. Die für diese unterschiedlichen Spaltungen verantwortlichen Sekretasen (α-, und vor allem β- und gamma-Sekretase) bilden daher auch primäre Angriffsziele einer möglichen AE Behandlungsstrategie. Es wurde daher versucht, Modulatoren bzw. Inhibitoren für diese Enzyme bei der Behandlung von AE einzusetzen (wie z.B. Benzodiazepine, Sulphonamide, Benzocaprolactame).

Ein weiteres Gen, das mit AE assoziiert ist, ist Apolipoprotein E, wobei hierfür drei allele Varianten existieren (APOE2, APOE3 und APOE4). Es hat sich gezeigt, dass Personen mit ein oder zwei Kopien von APOE4 ein höheres Risiko für AE aufweisen, wohingegen APOE2-Träger ein geringeres Risiko, verglichen mit der Gesamtbevölkerung aufweisen. Auch zeigt es sich, dass Personen, die Statine, also Arzneimittel, die Cholesterinbiosynthese inhibieren, zu sich nehmen, ein deutlich verringertes Risiko für AE aufweisen. Eine weitere Strategie zur Behandlung von AE konzentriert sich daher auf die Inhibition der Cholesterinbiosynthese, eben mit beispielsweise Statinen.

Ein weiterer Ansatz zur Behandlung von AE betrifft die Inhibition der Amyloid-Aggregation in zerebralen Plaques, welche unter anderem ebenfalls durch Sekretase-Inhibitoren durchgeführt werden könnte. Weiters wurde auch vorgeschlagen, den Zinkgehalt zu senken, da Zink in physiologisch relevanten Konzentrationen die Aggregation von Aß induzieren kann.

Schließlich wurden auch immunologische Strategien beschrieben, beispielsweise eine Immunisierung mit Aβ₄₂, welche jedoch im Rahmen einer klinischen Studie wegen schwerer Nebenwirkungen eingestellt werden musste (Willke, Bild der Wissenschaft, 9 (2003), 24-28).

Weitere AE-Behandlungsstrategien, die im Stand der Technik vorgeschlagen worden sind, betreffen die Verhinderung der APP-Expression und die Erhöhung der Aß-Clearance, wobei für erstere Substanzen gesucht wurden, die mit der APP-Promoter-Region wechselwirken. Im Hinblick auf die Aβ**-**Clearance wurde eine Erhöhung der Aktivität von bestimmten Proteasen, wie das Insulin abbauende Enzym und Neprolysin, oder die periphere Applikation von anti-Aβ-Antikörpern (De Mattos et al., PNAS 98 (15)(2001), 8850-8855) vorgeschlagen. Schließlich wurde auch versucht, bereits bestehende Amyloid-Plaques wieder aufzulösen, beispielsweise durch Erniedrigung des Amyloid β-Niveaus im Serum von AE-Patienten. In diesem Zusammenhang wurde auch vorgeschlagen, die Plaque-Ablagerungen von β-Amyloid-Proteinen im Gehirn durch Apherese-Verfahren zu reduzieren (US 6 551 266, worin die Entfernung von Makromolekülen mit einem Molekulargewicht von mehr als 500kD durch Apherese vorgeschlagen wird), ohne dass dies allerdings für AE auch tatsächlich gezeigt wurde. Die Auflösung durch bereits bestehende Plaques in Hirnzellen ist aber durch Aphereseverfahren direkt nicht möglich (Blut/Hirn-Schranke ist unüberwindlich für Plaques oder auch Moleküle mit >500kD).

Aufgabe der vorliegenden Erfindung war daher die Bereitstellung einer neuen Behandlungs- und Präventionsstrategie für die Alzheimer'sche Erkrankung.

Demgemäß wird mit der vorliegenden Erfindung eine Apherese-Vorrichtung, umfassend einen mit dem Blut oder Plasmafluss kontaktierbaren festen Träger, wie sie beispielsweise aus der Druchschrift US-A-4 770 774 bekannt ist, der einen Amyloid-β-Precurser-Protein (APP) bindenden Rezeptor aufweist, zur Verfügung gestellt. Mit der vorliegenden Apherese-Vorrichtung kann gezielt bei AE-Patienten bzw. Personen mit AE-Risiko mittels Apherese eine Clearance von APP oder APP-Abbauprodukten, insbesondere Aβ₄₀ oder Aβ₄₂, vorgenommen werden. Es ist bekannt, dass ein dynamisches Äquilibrium von Aβ₄₂ zwischen dem zentralen Nervensystem (ZNS) und dem Plasma besteht. Es konnte im Mausmodell gezeigt werden (DeMattos PNAS 2001, siehe oben), dass die periphere Applikation von anti-Aß-Antikörpern die ZNS und Plasma Aβ₄₂ Clearance beeinflusst und die Aβ₄₂ Belastung im Gehirn reduziert, ohne dass die anti-Aß-Antikörper die Blut-Hirn-Schranke überwinden. Diese Ergebnisse wurden von Matsuoka et al (Journal of Neuroscience 2003: 29-33) durch die periphere Applikation anderer Aβ₄₂ bindender Moleküle (Gelsolin und G_{M1}) bestätigt. Der Prozess der Entstehung der Plaques im Gehirn kann also durch Abfangen von Aβ₄₂ im Blut unterbunden werden. Hierbei ist es nicht kritisch, ob die Rezeptoren in der Apherese-Vorrichtung, die mit dem Blut oder Plasma des Patienten kontaktiert werden, spezifisch für Aβ₄₂ oder andere Abbauformen von APP sind, wesentlich ist nur, dass mit dieser spezifischen Adsorption APP und dessen (proteolytische) Abbauprodukten, insbesondere Aβ₄₂, aus dem Blut eliminiert werden, so dass es nicht zu einem "falschen" Proteinabbau (nämlich zu Aβ₄₂) kommt. Damit beruht die vorliegende Erfindung auf einem völlig anderen Anwendungsansatz für die Apherese als die US 6 551 266, nämlich auf der Eliminierung der potenziellen Plaque-Bausteine und nicht erst der Plaques. Im Übrigen scheidet die Eliminierung von Plaques mittels Apherese von vornherein als nicht effektiv zur AE-Behandlung aus, da die Blut-Apherese die Regionen der Plaqueentstehung im Gehirn gar nicht erreichen kann.

Auf der anderen Seite hat die erfindungsgemäße Apherese gegenüber Verfahren, die im Körper selbst zur Abreicherung von Aß führen (wie z.B. in DeMattos et al., PNAS 98(15)(2001), 8850-8855 mit peripheren anti-Aß Antikörpern), den entscheidenden Vorteil, dass hierbei keine Autoimmunantworten ausgelöst werden können. Weiters müssen erfindungsgemäß auch keine Substanzen dem Patienten zugeführt werden, die erst im Körper selbst wirken können (eventuell erst, nachdem sie an eine bestimmte Stelle transportiert worden), sondern das pathogene Agens wird gezielt entfernt, also die Ursache der Erkrankung spezifisch extrakorporal abgetrennt, ohne dass die Reaktionsprodukte im Körper eliminiert werden müssen.

Dabei können erfindungsgemäß die bereits bestehenden und bekannten Apherese-Vorrichtungen in allen Ausführungsformen leicht an die vorliegende Erfindung angepasst werden. Insbesondere sollte bei der Wahl des festen Trägers (und der Apheresevorrichtung) auf dessen (deren) medizintechnische Eignung Bedacht genommen werden. Derartige Träger, Verfahren oder Vorrichtungen sind unter anderem in der WO 97/48483 A, US 5 476 715, 6 036 614, 5 817 528 oder 6 551 266 beschrieben. Entsprechende kommerzielle Apherese-Apparate werden auch unter anderem von den Firmen Fresenius, Affina, Plasmaselect, ASAHI, Kaneka, Braun, etc. vertrieben, wie z.B. das LDL-Therasorb^{®}, das Immunosorba^{®}, das Prosorba®, das Globaffin^{®}, das Ig-Therasorb^{®}, das Immusorba^{®}, das Liposorba^{®}, das HELP^{®}, das DALI^{®}, das Bilirubin-Gallensäure-Absorber BR-350, die Prometheus^{®} Entgiftung, das MARS^{®}, das ADAsorb-System von Medicap oder das Plasma FLO-System. All diese Systeme - obgleich in der kommerziellen Form nicht primär immer auf die spezifische Eliminierung eines einzelnen Proteins gerichtet - können ohne weiteres von einem Apherese-Fachmann an die vorliegende Erfindung angepasst werden, z.B. als Immunapherese und/oder unter Einbau des erfindungsgemäßen festen Trägers (z.B. als Säule) in das Apherese-Gerät.

Unter "APP bindenden Rezeptoren" werden daher erfindungsgemäß auch sämtliche Substanzen verstanden, die eine Affinität zum Liganden APP und dessen biologischen Nebenprodukten, insbesondere Aβ₄₂, haben und in der Lage sind, diese Polypeptide aus dem Blut oder Plasma von AE-Patienten oder Personen mit einem Risiko für AE zu entfernen. Diese APP- bzw. Aβ₄₂-Rezeptoren können dabei vorzugsweise (poly oder monoklonale) Antikörper, Proteine, Peptide, Ganglioside oder Nukleinsäuren sein.

Besonders bevorzugt sind dabei anti-APP-Antikörper, anti-Aß40-Antikörper oder anti-Aβ₄₂-Antikörper, APP bindende Proteine , insbesondere Gelsolin, apoJ oder apoE, APP bindende Peptide, APP bindende Ganglioside, insbesondere G_{M1}, oder APP bindende Nukleinsäuren, insbesondere Aptamere) oder Mischungen dieser Rezeptoren.

Beispiele für derartige Antikörper sind 3D6 (Aβ₁₋₅), 2H3 (Aβ₁₋₁₂), 2G3 (Aβ₃₃₋₄₀), 21F12 (Aβ₃₃₋₄₂), 12H7 (Aβ₃₃₋₄₂) (Johnson-Wood et al, PNAS 1997:1550-1555), 10D5, 16C11 (Bard et al, Nature Medicine 2000:916-919) die De Mattos et al. (2001) beschriebenen Antikörper (m266, m243) sowie Antikörper gleicher Spezifität. Derartige Antikörper werden beispielsweise bei der Immunisierung von Säugetieren mit Vakzinformulierungen, enthaltend APP, Aß₄₂ oder Fragmente oder Varianten davon, erhalten, gegebenenfalls gefolgt von Zellfusion und Klonselektionsprotokollen (bei monoklonalen Antikörpern).

Gelsolin (Matsuoka et al 2003, siehe oben), apoJ und apoE (DeMattos et al 2001, siehe oben) sind weitere Beispiele für APP-bindende Protein-Rezeptoren. G_{M1} ist ein Beispiel für einen APP-bindenden Gangliosid-Rezeptor (Matsuoka et al 2003, siehe oben) .

Weitere Beispiele für APP-bindende Proteine sind das CETP (Cholesteryl-Ester-Transfer-Protein) und das ERAB-Protein (261 AS groß; He et al., JBC 273 (17) (1998), 10741-10746; Lustbader et al., Science 304 (2004), 448-452; insbesondere AS 1-186 bzw. 1-158). Beispiele für APP-bindende Peptide sind die aus den APP-bindende Proteinen abgeleiteten APP-bindenden Fragmente. Konkrete Beispiele für APP-bindende Peptide sind KTYNLKKGQT-C ("Peptid 4077"), GIAVASKTYNLKKGQTHTLEDFQRVLDV (ERAB 93-120), SKTYNLKKG-QTHT (ERAB 98-110), C-HQKLVFFAED ("Peptid 1323"), C-EVHHQKLVFFA-EDVGS ("Peptid 1324"), C-HQKIVFFAED ("Peptid 1325") und FGFPEHLLVDFLQSLS-C ("Peptid 1208") (bzw. auch alle anderen ERAB, CETP oder β-breaker Fragmente, die die APP-bindenden Bereiche beinhalten) (die terminalen Cysteine sind bei diesen Peptiden nicht Teil der nativen Proteinsequenz sondern wurden zu Kopplung der Peptide angehängt).

Peptide als APP-bindende Rezeptoren können dabei aus D- oder L-Aminosäuren oder Kombinationen von D und L-Aminosäuren zusammengesetzt sein, und gegebenenfalls durch weitere Modifizierungen, Ringschlüsse oder Derivatisierungen verändert worden sein. Geeignete Peptidrezeptoren für z.B. Aβ₄₂ können aus Peptid-bibliotheken zur Verfügung gestellt werden, die kommerziell erhältlich sind. Vorzugsweise sind diese Peptide zumindest 5, vorzugsweise 6, Aminosäuren lang, insbesondere mindestens 8 Aminosäuren, wobei bevorzugte Längen sich bis zu 11, vorzugsweise bis zu 14 oder 20 Aminosäuren erstrecken können. Erfindungsgemäß können jedoch auch längere Peptide ohne weiteres als APP bindende Rezeptoren herangezogen werden. Desweiteren eignen sich Oligomere (wie z.B. Polyethylenimin und Polylysin) als Rezeptoren.

Zur Herstellung derartiger APP-bindender Rezeptoren sind selbstverständlich auch Phagen-Bibliotheken, Peptid-Bibliotheken, z.B. mittels kombinatorischer Chemie erzeugt oder mittels high throughput screening-Techniken für verschiedenste Strukturen, geeignet (siehe z.B. in Phage Display : A Laboratory Manual by Carlos F. Barbas (Editor), et al; Willats WG, Phage display: practicalities and prospects. Plant Mol Biol. 2002 Dec; 50(6): 837-54; http://www.microcollections.de/showpublications.php#). Neben Phagenbibliotheken auf Randomisierungsbasis eigen sich ebensolche Bibliotheken, die sich des ribosomalen Displays oder des bakteriellen Displays bedienen. Entsprechende Bibliotheken und Verfahren zu ihrer Generierung sind dem Fachmann bekannt und unter anderem in US 2004/0110281 A, WO 00/72880 A und WO 02/059148 A beschrieben.

Weiters können auch APP-bindende Rezeptoren auf Basis von Nukleinsäuren ("Aptamere"; aber auch "Decoy"-Oligodeoxynuleotide (ds Oligonukleotide, die von der Sequenz her Bindungsstellen für Transkriptionsfaktoren darstellen)) eingesetzt werden, wobei auch diese mit verschiedensten (Oligonukleotid-) Bibliotheken (z.B. mit 2-180 Nukleinsäureresten) aufgefunden werden können (z.B. Burgstaller et al., Curr. Opin. Drug Discov. Dev. 5 (5) (2002), 690-700; Famulok et al., Acc. Chem. Res. 33 (2000), 591-599; Mayer et al., PNAS 98 (2001), 4961-4965, uvm.). Das Nukleinsäurerückgrat kann dabei beispielsweise durch die natürlichen Phosphordiester-Verbindungen aber auch durch Phosphorotioate oder Kombinationen oder chemische Variationen (z.B. als PNA) aufgefunden werden, wobei als Basen erfindungsgemäß vor allem U, T, A, C, G, H und mC eingesetzt werden können. Die 2'-Reste der Nukleotide, die gemäß der vorliegenden Erfindung eingesetzt werden können, sind vorzugsweise H, OH, F, Cl, NH₂, O-Methyl, O-Ethtyl, O-Propyl oder O-Buthyl, wobei die Nukleinsäuren auch noch anders modifiziert werden können, also beispielsweise mit Schutzgruppen, wie sie üblicherweise in der Oligonukleotidsynthese angewendet werden, versehen werden. APP-bindende Aptamere sind daher ebenfalls bevorzugte APP-bindende Affinitätsmoleküle im Rahmen der vorliegenden Erfindung.

Die Aptamere für APP findet man bespielsweise mit dem nachfolgend beschriebenen Verfahren. Man kontaktiert immobilisiertes APP (oder eine andere der oben beschriebenen Formen) mit einer Mischung aus Nukleinsäuren, wobei hochaffin bindende Nukleinsäuren von den weniger affin oder gar nicht bindenden Nukleinsäuren abgetrennt werden. Bei den Mischungen mit Nukleinsäuren handelt es sich typischerweise um Nukleinsäurenbibliotheken, welche beispielsweise im Wege der kombinatorischen Chemie hergestellt wurden. Eine Nukleinsäurebibliothek enthält eine Vielzahl voneinander unterschiedlicher Nukleinsäuren, wobei zumindest in einem Teilsequenzbereich eine Randomisierung (mit natürlichen und/oder nicht-natürlichen Nukleotiden) eingerichtet ist. Es kann, muss aber nicht, ein konservierter Sequenzbereich vorgesehen sein. Randomisierung in n Positionen mit m verschiedenen Nukleotiden führt zu einer Bibliothek mit n^{m} Elementen.

Für das Auffinden der APP-spezifischen Affinitätsnukleinsäuren werden folgende Schritte durchgeführt. a) eine Säule wird (innenseitig) mit APP (etc.) beladen und APP (etc.) wird in der Säule immobilisiert, b) das Gemisch an Nukleinsäuren wird einem ersten Ende der Säule aufgegeben, wobei durch die Säule ein definierter Volumenstrom an Trägersubstanz, laufend vom ersten Ende zum zweiten Ende der Säule, eingerichtet ist, c) die Nukleinsäuren werden mit abnehmender Affinität der Nukleinsäuren zu APP (etc.) in zunehmendem Abstand vom ersten Ende der Säule gebunden und immobilisiert, d) der Volumenstrom an Trägersubstanz durch die Säule wird nach einer definierten Laufzeit beendet, e) die Säule wird durch eine Mehrzahl von Teilungen in Säulensegmente aufgetrennt, wobei jedem Segment eine Laufwegkoordinate zugeordnet wird, f) aus zumindest einem Segment werden die immobilisierten Nukleinsäuren unspezifisch desorbiert und unter Zuordnung der dem Segment zugeordneten Laufwegkoordinate gewonnen. Der Ausdruck innenseitig der Säule meint innerhalb eines Lumens in aller Allgemeinheit. Der Ausdruck der Säule sollte alle Arten fester Trägersysteme umfassen können, z.B. auch nicht vollständig umschlossene Trägersysteme sind möglich.

Die Immobilisierung von APP (etc.) kann nach den üblichen Methoden der Säulenchromatographie erfolgen. Als Säule ist jedes mechanisches Konstrukt bezeichnet, welches ein Lumen mit zwei Enden aufweist. Als Strukturmaterial kommen alle für Säulen üblichen Materialien, wie Metalle, Glas und/oder Kunststoffe in Frage. Die Säule kann innenseitig mit einer APP-(etc.) bindenden Matrix versehen sein und/oder das Strukturmaterial kann zur direkten Bindung der Targetmoleküle geeignet oder vorbereitet sein. Ein Gemisch aus Nukleinsäuren bezeichnet Nukleinsäurebibliotheken mit einer Anzahl von typischerweise 10⁶ bis 10²²/Mol, insbesondere 10¹⁰ bis 10²¹/Mol, voneinander verschiedener Nukleinsäurenspezies. In der auf die Säule aufgetragenen Bibliothek ist jede Nukleinsäurespezies statistisch beispielsweise mit 10 bis 10¹⁷, insbesondere 100 bis 10¹³, Molekülen vertreten. Die Trägersubstanz ist üblicherweise eine Flüssigkeit, in welcher die Nukleinsäurebibliothek löslich und stabil ist. Hierfür kommen alle für Nukleinsäurebibliotheken üblichen Puffer und dergleichen in Frage. Der Volumenstrom an Trägersubstanz kann vor Auftrag der Nukleinsäurebibliothek eingestellt werden. Dann wird die Nukleinsäurebibliothek säuleneingangsseitig dem Trägersubstanzstrom zugegeben. Die Nukleinsäurebibliothek kann aber auch unmittelbar aufgegeben werden. Nach einer Laufzeit, welche durch den Aufbau der Säule und den eingestellten Volumenstrom bestimmt ist, tritt der "Pfropfen", welcher durch die Nukleinsäurebibliothek aufgegeben wurde, säulenausgangsseitig wieder aus (verbreitert durch Faltung mit der Diffusion), wobei gebundene Nukleinsäuren aus dem "Pfropfen" abgetrennt und in der Säule immobilisiert wurden. Zweckmäßigerweise wird der Volumenstrom durch die Säule wenig- bzw. nicht-turbulent, vorzugsweise laminar, eingestellt (Summe der Beschleunigungsvektoren der Trägersubstanz über das Säulenvolumen, insbesondere über den Säulenquerschnitt, minimal, idealerweise 0). Die Gesamtanzahl der APP-Moleküle in der Säule beträgt typischerweise das 10²- bis 10¹⁶-fache, insbesondere das 10³- bis 10¹⁵-fache, der Anzahl an Nukleinsäuremolekülen einer einzelnen Spezies in der aufgetragenen Nukleinsäurebibliothek. Die Bindung der Nukleinsäuren an die APP-Moleküle erfolgt vorzugsweise unter Bedingungen, welche einem späteren Einsatz der Nukleinsäuren bei der Apherese entsprechen, beispielsweise in einem geeigneten Puffer, welcher entsprechend abgestimmt ist hinsichtlich Temperatur, Ionenstärke, pH-Wert und Pufferbedingungen. Die Trägersubstanz sowie das Lösungsmittel der Nukleinsäurenbibliothek sind dann entsprechend hinsichtlich derer Komponenten auszuwählen. Die Teilung der Säule in eine Mehrzahl von Säulensegmente kann beispielsweise durch Zerschneiden der Säule erfolgen, wobei die Schnitte vorzugsweise orthogonal zum Volumenstromvektor erfolgen. Die Säule kann aber auch zuvor aus Säulensegmenten zusammengesetzt worden sein, wobei sich ein Säulensegment vorzugsweise in Richtung des Volumenstromvektors an das nächste Säulensegment dicht aneinanderreiht (Fügequerschnitt orthogonal zum Volumenstromvektor). Dann kann die Teilung durch Auflösung des zuvor gebildeten Verbundes aus Säulensegmenten erfolgen. Die unspezifische Desorption kann durch Eluierung mit einem hinreichend starken Liganden durch Verdrängung, Komplexierung, Modifikation und/oder Zerstörung von APP, physikochemisch oder thermisch erfolgen. Auch mechanische Verfahren, beispielsweise Ultraschall, können zur Desorption oder zur Verstärkung der Desorption eingesetzt werden. Es können auch Kombinationen der vorstehenden Desorptionsverfahren angewandt werden. Es versteht sich, dass die Nukleinsäuren von dem verwendeten Desorptionsverfahren nicht zersetzt werden dürfen.

Die oben beschriebenen Schritte beruhen auf der Erkenntnis, dass eine Nukleinsäurebibliothek sich analog einer Proteinmischung mittels der Affinitätschromatographie räumlich nach Massgabe der Affinität zum Targetmolekül, im hier beschriebenen Fal, für APP (etc.), trennen lässt. Die Erfindung beruht weiterhin auf der Erkenntnis, dass eine mittels unspezifischer Desorption sich einstellende Vermengung desorbierender Nukleinsäuren verschiedener Affinität dadurch vermeidbar ist, dass vor der unspezifischen Desorption die Säule mit den darin gebundenen Nukleinsäuren gleichsam in Affinitätsabschnitte aufgeteilt und die in den so erhaltenen Affinitätsabschnitten bzw. Säulensegmenten gebundenen Nukleinsäuren sich leicht und ohne störende Ligandenkoppelungen beispielsweise im Rahmen einer PCR oder RT-PCR unspezifisch desorbieren und ebenso unspezifisch amplifizieren lassen. Subsequente Selektionsartefakte werden vermieden. Ebenso wenig sind Liganden, insbesondere hohe Konzentrationen an Liganden, zur Desorption erforderlich. Schließlich stehen praktisch alle gebundenen und dann desorbierten Nukleinsäuremoleküle für eine Amplifikation zur Verfügung. Dies erlaubt es, mit geringen Nukleinsäurekonzentrationen zu arbeiten. Es ist grundsätzlich bereits ausreichend, wenn in der Nukleinsäurebibliothek jede Spezies im statistischen Mittel mit einem Molekül vertreten ist. Wenn die Anzahl der APP-Moleküle in einem Säulensegment statistisch 1 beträgt, dann können sogar einzelne Nukleinsäurespezies nach ihrer Affinität zum Targetmolekül getrennt werden.

Ein besonderer Vorzug dieses Verfahrens ist nachfolgend erläutert. Die Auftrennung der Nukleinsäuren nach Affinität führt dazu, dass Nukleinsäuren in einem Säulensegment eine ähnliche Affinität bei unterschiedlicher Spezifität (unterschiedliche Regionen von APP werden gebunden bzw. sind Bindungsstellen für die APP-Affinitätsnukleinsäuren) aufweisen.

Grundsätzlich reicht es aus, wenn ein Segment, welchem eine gewünschte Affinität zugeordnet ist, (isoliert) zur Desorption weiterverarbeitet wird. Dies setzt allerdings - außer bei maximaler Affinität, wobei das, bezogen auf den Volumenstromvektor, erste Säulensegment weiter verarbeitet wird - eine Vorstellung über die Affinitätsverteilung bei der aufgetragenen Nukleinsäurebibliothek voraus. Bevorzugt ist es daher in der Regel, dass die immobilisierten Nukleinsäuren aus jedem Segment separat desorbiert und gewonnen werden unter jeweiliger Zuordnung der Laufwegkoordinaten jeden Segments zu den daraus gewonnenen Nukleinsäuren.

Grundsätzlich ist jede Art der Desorption möglich. Vorzugsweise wird die unspezifische Desorption mittels üblicher physikochemischer oder thermischer Verfahren durchgeführt wird. Thermische Desorption erfolgt durch Erwärmung des Säulensegments bzw. der darin enthaltenen Lösung. Die Erwärmung kann beispielsweise durch elektrische Beheizung oder Einstrahlung von Mikrowellen oder IR erfolgen. Insbesondere sind die Erwärmungstechniken aus der PCR Technologie geeignet. Neben der Amplifikation von Nukleinsäuren bzw. Aptameren mittels Polymerase können selbstverständlich auch andere Amplifikationsverfahren, wie beispielsweise mittels Ligase, angewandt werden. Die unspezifische Desorption kann durch chemische Modifikation von APP unterstützt werden, z. B. Oxidation durch Natriumperjodat oder dergleichen, oder durch unspezifische Komplexbildung, z. B. mittels Borat oder dergleichen zur Blockierung von cis-trans-Diolbindungen in Kohlenhydraten.

Daher ist es bevorzugt, wenn die unspezifische Desorption durch thermische Desorption in einer, vorzugsweise verlängerten Hochtemperaturphase einer PCR oder RT-PCR durchgeführt wird. Hierbei wird ein Synergieeffekt erzielt, da in aller Regel, insbesondere beim Arbeiten mit Nukleinsäurebibliotheken mit niedrigen Konzentrationen der Nukleinsäurespezies, eine Amplifizierung ohnehin erforderlich ist. Zur Erhöhung der Ausbeute wird beispielsweise mit 5 bis 60, vorzugsweise mit 20 bis 60, höchstvorzugsweise mit 45 bis 55, Zyklen gearbeitet. Im Rahmen der Amplifikation kann mit mindestens einem markierten Primer gearbeitet werden. Der Primer kann mindestens eine Endonukleaseschnittstelle aufweisen. Eine solche Schnittstelle dient beispielsweise dazu, das Amplifikat von größeren Bereichen der Primersequenz zu befreien. Nukleotidbausteine, sei es im Primer oder bei den zu selektierenden Nukleinsäuren, können beispielsweise mittels Fluoreszenzfarbstoffen markiert sein. Als Fluoreszenzfarbstoffe seien beispielsweise genannt: Alexa TM Fluor 488, Fluor 532, Fluor 546, Fluor 568, Fluor 594, Oregon Green 488, Fluorescein, Rhodamine 6G, Tetramethylrhodamine, Rhodamine B und Texas Red. Das Amplifikat kann auch durch zwei verschiedene chemische Modifikationen an verschiedenen Enden markiert sein, sofern die bei der Modifikation eingeführten Gruppen geeignet sind, als Liganden jeweils an einer unterschiedlichen Affinitätsmatrix gebunden werden zu können.

Es empfiehlt sich, zur sicheren Abtrennung nicht-affiner oder niedriger-affiner Nukleinsäuren zwischen geeigneten Verfahrenstufen Waschverfahrensschritte einzufügen. Insbesondere ist es bevorzugt, wenn zwischen den Verfahrensschritten d) und e) zumindest ein Waschverfahrensschritt durchgeführt wird. Zum Waschen ist beispielsweise das Lösungsmittel bzw. das Medium der Nukleinsäurebibliothek bzw. die Trägersubstanz geeignet.

Bevorzugt ist es, wenn die innenseitige Belegung der Säule mit APP (etc.) und dessen Immobilisierung mittels kovalenter Bindung, vorzugsweise nach Aktivierung mit chemisch hochreaktiven Gruppen (beispielsweise Tresylchlorid, Cyanbromid und/oder Periodat) oder über bifunktionale Spacerverbindungen nach Modifikation mit chemisch wenig reaktiven Gruppen (beispielsweise Amin, Hydroxy, Keto und/oder Carboxyl), durchgeführt wird. Beispiele für Spacergerüste geeigneter Spacerverbindungen sind: substituierte und unsubstituierte C2-C10 Alkylgruppen, substituierte und unsubstituierte C2-C10 Alkenylgruppen, substituierte und unsubstituierte C2-C10 Alkynylgruppen, substituierte und unsubstituierte C4-C7 Carbocylo alkylgruppen, substituierte und unsubstituierte C4-C7 Carbocylo alkenylgruppen, substituierte und unsubstituierte C7-C14 Aralkylgruppen, ein heterocyclisches Molekül mit Heteroatomen ausgewählt aus Stickstoff, Sauerstoff, Schwefel, wobei besagte Substitutionen bestehen können aus Alkyl, Alkenyl, Alkynyl, Alkoxy, Thiol, Thioalkoxy, Hydroxyl, Aryl, Benzyl, Phenyl, Nitro, Halogen, Äthergruppen mit 2 bis 10 Kohlenstoffatomen und 1 bis 4 Sauerstoff oder Schwefel Atomen, Polyalkyl glycol, Halogen, Hydroxyl, Thiol, Keto, Carboxyl, Amide, Ätherverbindungen, Thioäther, Arnidinederivaten, Guanidinederivaten, Glutamylderivaten, Nitrat (ONO₂), Nitro (NO₂), Nitril, Trifluoromethyl (-CF3), Trifluoromethoxy (-OCF₃), O-Alkyl, S-Alkyl, NH-Alkyl, N-Dialkyl, O-Aralkyl, S-Aralkyl, NH-Aralkyl, Amino, Azido (N₃), Hydrazino (NHNH₂), Hydroxylamino (ONH₂), Sulfoxide (SO), Sulfone (SO₂), Sulfide (S-), Disulfide (S-S), Silyl bestehen kann. Spacerverbindungen sind typischerweise bifunktional, wobei die Funktionalitäten gleich oder verschieden sein können und beispielsweise ausgewählt sind aus der Gruppe bestehend aus "N-Hydroxysuccinimid und Hydrazide".

Zur Verringerung der Varietät innerhalb der aus einem Säulensegment erhältlichen Nukleinsäuren ist es bevorzugt, wenn jedes Säulensegment im statistischen Mittel 0,1 bis 10³, vorzugsweise 1 bis 10², höchstvorzugsweise 1 bis 10, APP (etc.)-Moleküle enthält. Hierauf abgestimmt kann die Nukleinsäurebibliothek, wie aufgetragen, im statistischen Mittel 0,1 bis 10³, vorzugsweise 1 bis 10², höchstvorzugsweise 1 bis 10, Nukleinsäuremoleküle einer Spezies enthalten.

Für das Strukturmaterial der Säule kommen grundsätzlich alle beispielsweise aus der Affinitätschromatographie bekannten Werkstoffe in Frage. Hierzu gehören Säulen aus Kieselgel oder Polymere, wie Polyethylen nach Aktivierung durch chemische Derivatisierung oder Plasmaaktivierung. Die Länge der Säulensegmente liegt zweckmäßigerweise im Bereich von 0,1 µm bis 1 mm, vorzugsweise 0,1 bis 100 µm, höchstvorzugsweise 0,5 bis 10 µm. Solche Schnitte lassen sich unschwer beispielsweise mittels eines Mikrotoms herstellen. Der Innendurchmesser der Säule liegt zweckmäßigerweise im Bereich von 0,05 bis 1 mm, vorzugsweise von 0,1 bis 0,5 mm, höchstvorzugsweise von 0,2 bis 0,4 mm.

Zweckmäßig ist es, wenn vor der eigentlichen Trennung (Bindung oder mechanische Auftrennung) der Nukleinsäuren unerwünschte Nukleinsäuren eliminiert werden. Unerwünschte Nukleinsäure sind beispielsweise Nukleinsäuren, welche an APP-freie Innenoberflächen der Säule binden. Dann kann eine APP-freie Säule vorgeschaltet und die Nukleinsäurebibliothek zuvor hierdurch geleitet werden.

Bei den vorstehenden Verfahrensweisen kann kontinuierlich, i. e. beispielsweise durch Hintereinanderschalten von Säulen, oder diskontinuierlich, beispielsweise durch zwischenzeitliches Auffangen des Eluenten aus der Vorsäule, gearbeitet werden.

Zur Erzielung einer weiteren Verbesserung der Affinitätstrennung kann grundsätzlich auf verschiedene Weisen verfahren werden. So können die aus einem oder mehreren Segmenten desorbierten Nukleinsäuren, ggf. nach Amplifikation wiederholt den erfindungsgemäßen Verfahren unterworfen werden, und/oder die Elutropie der Bedingungen bei der Desorption kann erhöht werden (Temperatur, Ionenstärke, pH-Wert, Pufferbedingungen). Alternativ kann die Raumdichte von APP und folglich der gebundenen Nukleinsäuren, i. e. die Anzahl der APP-Moleküle, in einem Säulensegment verringert werden.

APP-bindende Aptamere (die erfindungsgemäß wie oben definiert auch Aß₄₂ bindende Aptamere miteinschließen) sind daher ebenfalls bevorzugte APP bindende Rezeptoren im Rahmen der vorliegenden Erfindung.

Erfindungsgemäß werden daher die APP-bindenden Rezeptoren, die vorzugsweise aus Peptiden, Antikörpern oder Nukleinsäuren bestehen, an ein geeignetes Trägermaterial zur extra-korporalen Eliminierung von APP und dessen proteolytischen Abbauprodukten in Alzheimer-(Risiko-)Patienten verwendet.

Bei der Anwendung der vorliegenden Erfindung in medizinischer Routinepraxis ist es erforderlich, dass der Träger steril und pyrogenfrei ist, so dass jede Trägersubstanz bzw. jede Rezeptor-/Träger-Kombination, die diese Merkmale erfüllt, erfindungsgemäß bevorzugt ist (siehe z.B. US 6 030 614 oder US 5 476 715). Zu den geeigneten Beispielen zählen poröse Homopolymere, Co- oder Terpolymere von Vinyl enthaltenden Monomeren (z.B. Acryl-Säure, wie z.B. TSK Toyopearl, Fractogel TSK), Träger mit Modifkationen (Aktivierungen) mit Oxiran enthaltenden Verbindungen (z.B. Epichlorohydrin) und gegebenenfalls weiteren Reaktionen mit NH₃, Amino oder Carboxyl enthaltenden Verbindungen, oder CNBr oder CNCl-Adsorbientien, wie in der EP 110 409 A und der DE 36 17 672 A beschrieben. Besonders bevorzugte Adsorptionsmaterialien für therapeutische Zwecke sind geeignet, einen Verlust von Blutzellen zu vermeiden, aktivieren das Komplementsystem nicht oder nur geringfügig und halten eine Aggregatbildung im extra-korporalen Kreislauf möglichst hintan. Weiters sollten die eingesetzten Trägermaterialien vorzugsweise auch in rezeptorgekoppelter Form ausreichend stabil gegenüber Sterilisierungsmaßnahmen sein, insbesondere gegenüber EthylenOxid-Sättigung, Glutaraldehyd-Sättigung, Gamma-Bestrahlung, Dampfbehandlung, UV-Behandlung, Lösungsmittelbehandlung und/oder Detergensbehandlung, etc.. Beispielsweise können auch Produkte auf Sepharose-, Agarose-, Acryl-, Vinyl-, Dextran- etc. -Basis eingesetzt werden, die vorzugsweise geeignete funktionelle Gruppen zur Anbindung der APP bindenden Rezeptoren bereits kommerziell erhältlich aufweisen. Weitere geeignete Träger schließen auch Monolithe ein (Träger auf Basis von quervernetzten Glycidylmethacrylat-co-ethylenglykoldimethacrylat-Polymer) Subpol (Poschalko et al., J Am Chem Soc. 2003 Nov 5; 125(44): 13415-26.).

Zur Kopplung der Rezeptoren an die geeigneten Träger ist die dem Fachmann bekannte Chemie einsetzbar (z.B. Bioconjugate Techniques, Greg T Hermanson, Ed., Academic Press, Inc, San Diego, CA, 1995, 785pp).

Gemäß einem weiteren Aspekt betrifft die vorliegende Erfindung die Verwendung der erfindungsgemäßen Vorrichtung zur Bereitstellung einer Behandlungsvorrichtung zur Behandlung der Alzheimer'schen Erkrankung oder zur Vorbeugung einer derartigen Erkrankung, indem die Vorrichtung geeignet zur Behandlung des jeweiligen Patienten hergerichtet wird. Bei der Durchführung der Behandlung wird ein Patient für eine zur effektiven Eliminierung von APP-Polypeptiden ausreichende Zeitdauer an die Apherese-Apparatur angeschlossen, wobei der Blut- oder Plasmafluss des Patienten mit dem festen Träger, umfassend den APP bindenden Rezeptor, kontaktiert wird, worauf APP und/oder die proteolytischen Abbauprodukte von APP, insbesondere Aβ₄₂, gebunden werden. Im Zuge der Apherese-Behandlung sind natürlich peripherer oder zentralvenöser Venenzugang bzw. arterievenöse Fistel sicherzustellen, ebenso wie ausreichende Antikoagulation, sowie die erforderlichen Quantifizierungs- und Messdaten aufzuzeichnen. Weiters wird bei den meisten Apherese-Verfahren eine Primär-Trennung von Plasma und Blutzellen vor der eigentlichen Plasmabehandlung erforderlich sein. Besondere Personen, bei denen eine vorbeugende Maßnahme erforderlich ist, sind familiär belastete Personen, ältere Personen (>50, >60 oder >70 Jahre) oder Personen mit einem anderen Risikofaktor für AE, insbesondere genetische Faktoren.

Die Erfindung wird anhand der nachfolgenden Beispiele sowie der Zeichnungsfiguren, auf die sie selbstverständlich nicht eingeschränkt ist, näher erläutert.

Es zeigen:
Fig. 1: die Bindung von Aβ₄₂ an das Peptid 1208 (gekoppelt an BSA und an ELISA Platte ge-coated);
Fig. 2: die Bindung von Peptid 1208 (und 1208-BSA) an Aβ₄₂ (ge-coated an ELISA Platte);
Fig. 3: die kompetitive Bindung von Aβ₄₂ an 1208-BSA.

### 1. Herstellung des APP-Rezeptor tragenden Trägers

### 1.1 Monolithische Säule

Eine CIM^{®} Epoxy Monolithic Säule (BIA Separations, SI) wird gemäß den Angaben des Herstellers mit 0,5 M Na-Phosphat-Puffer bei pH 8,0 äquilibriert und ein monoklonarer Antikörper gegen Aß Peptid ebenfalls gemäß Herstellerangaben aktiviert und an die CIM-Säule gekoppelt. Die Säule wird mehrfach mit Phosphat-Puffer gewaschen (+ 1 M NaCl) und überschüssige Epoxy-Gruppen gegebenenfalls noch blockiert.

Qualitätssicherung wird mittels Kontrolle im Wasch- und Äquilibrationseluat durchgeführt; nur Säulen ohne aktive Epoxy-Gruppen und ohne Antikörper-Leakage im Eluat werden weiterverwendet und in eine Apharese-Apparatur eingebaut.

### 1.2 Sepharose-Säule

Ein Agarose Bulk-Material (Sepharose CL4B) wird aseptisch in einen sterilen und pyrogenfreien Behälter gefüllt und das Material aseptisch gewaschen, wobei zwischen jedem Waschschritt das Gelmaterial völlig unter Vakuum getrocknet wird. Die Sepharose wird anschließend für 30 Minuten bei 115°C im Autoklaven dampfsterilisiert.

Nach der Sterilisierung wird die Sepharose in einem sterilen Behälter in 60% Aceton/Wasser aufgenommen und mit CNBr und Triethylamin aktiviert (14 g CNBr pro 96 ml Acton; 30 ml Triethylamin in 66,2 ml 87 %igem Aceton). Dann wurde eine Aceton/HCl-Lösung zugesetzt (392 ml steriles, pyrogenfreies Wasser; 16,3 ml 5 N HCl, 408 ml Aceton). Die aktivierte Sepharose wird gewaschen und innerhalb von 2 h der Kopplungsreaktion zugeführt, um die Hydrolyse von aktivierten Gruppen zu verhindern.

Eine sterilfiltrierte Antikörper-Lösung (m266 bzw. m243) wird in das Reaktionsgefäß eingebracht und für mindestens 90 min gerührt. Schließlich wird die Reaktionslösung gründlich gewaschen (mit isotonischem Phosphat-Puffer), bis keine Reaktionsprodukte im Eluat nachweisbar sind, und die Antikörper-gekoppelte Sepharose in sterile und depyrogenisierte Glassäulen mit Glassintern gefüllt und einer abschließenden Qualitätskontrolle unterzogen (Eluatanalyse hinischtlich Reaktionsprodukten, Schwermetallen etc.; Partikelanalyse, Pyrogenizität; Sterilität).

### 2. Tiermodell für die Apherese-Behandlung von Alzheimer-Patienten

Im Institut für Diabetes "Gerhardt Katsch" ist ein miniaturisiertes extrakorporales System zur Anwendung der Apherese-Therapie am Kleintiermodell Ratte entwickelt worden. Ein derar-tiges Apherese-Testsystem ist weltweit nur sehr begrenzt verfüg-bar. Die wiederholte Apherese-Behandlung beim gleichen Ver-suchstier und sich daran anschließende Untersuchungen in der Nachbeobachtungsphase zur Beurteilung des Therapieerfolges haben einen hohen Neuheitswert und sind im internationalen Schrifttum bisher nicht beschrieben worden.

Die Apherese ist ein alternatives Therapieverfahren, bei dem Blut außerhalb des Körpers krankheitsverursachende Substanzen entzogen werden. In der Humanmedizin ist die Apherese bisher zur Behandlung von mehr als 100 Krankheiten eingesetzt worden. Mit Hilfe der Apherese konnten krankheitsrelevante Substanzen u.a. bei Autoimmunerkrankungen wie rheumatoider Arthritis, Myasthenia gravis, endokriner Ophthalmopathie, Multipler Sklerose, systemischem Lupus erythematosus, Stiff-Man-Syndrom und Typ-1-Diabetes vollständig oder zumindest teilweise aus dem Blut entfernt werden. Bisher wird die Apherese jedoch nur als alternatives Verfahren zur Behandlung von Therapie-resistenten chronischen Erkrankungen, die mit starken Belastungen und einer deutlichen Einschränkung der Lebensqualität der betroffenen Patienten verbunden sind, anerkannt. Ein wesentlicher Grund dafür ist, dass bisher keine detaillierten Kenntnisse zu den Wirkmechanismen einer erfolgreichen Apherese-Therapie vorliegen.

Durch Verwendung anerkannter Tiermodelle, wie z.B. für den Typ-1-Diabetes und die rheumatoide Arthritis, können sowohl die Wirkmechanismen der Apherese-Verfahren weiter aufgeklärt als auch neuartige Indikationsstellungen für die Apherese-Behandlung präklinisch getestet werden. Für die Durchführung von Apherese-Behandlungen werden die Versuchstiere zunächst mit chronischen Gefäßkathetern versehen. Im extrakorporalen Kreislauf werden anschließend die plasmatischen und zellulären Bestandteile des Blutes über Plasmafilter getrennt. Während die zellulären Bestandteile sofort an das Tier zurückgehen, kann das Plasma vor der Rückführung durch verschiedene Adsorptionsverfahren (Immunadsorption o.a.) gereinigt werden. Die gute Verträglichkeit solcher wiederholter Apherese-Behandlungen ist bereits für verschiedene Rattenstämme belegt worden (Körpermasse, Hämatokrit, Allgemeinzustand). Das Apherese-Testsystem ist bei Tieren mit einem Mindestgewicht von 250g erprobt und an Ratten-Modellen für Autoimmunerkrankungen (Typ-1-Diabetes, Kollagen Typ II-induzierte Arthritis) eingesetzt worden.

Das extrakorporale System zur Plasmapherese am Kleintiermodell kann für verschiedene Arten der Adsorption herangezogen werden. Die Anwendung dieses Testsystems an Modellen für chronische Erkrankungen ermöglicht (A) die Erprobung neuer Behandlungs- und Präventionsstrategien, (B) die Entschlüsselung der Wirkmechanismen verschiedener Apherese-Technologien und (C) die Ermittlung der Indikationsstellung für einzelne Apherese-Therapieverfahren. Das IDK ist Ihr kompetenter Partner bei der präklinischen Testung neu entwickelter Apherese-Verfahren und kann wesentlich zur Überführung und Markteinführung neuer therapeutischer Verfahren von der Entwicklungsphase über die tierexperimentelle Erprobung bis hin zur klinischen Anwendung beim Patienten beitragen (http://www.praeklinik.de/).

Vor Beginn der experimentellen Apherese-Therapie werden die Tiere mit arteriellen und venösen Kathetern versehen, beziehungsweise es werden chronisch katheterisierte Ratten (Gefäßkatheter, die zur Applikation von Testsubstanzen und zur Blutproben-entnahme sowie zur Durchführung der Apherese-Therapie und der Clamp-Untersuchungen im Tiermodell geeignet sind) eingesetzt. In einem ersten Schritt werden bei der Apherese zunächst Blutzellen und Plasma mittels Plasmafilter getrennt. Während die Blutzellen unmittelbar in das Tier refundiert werden (über den venösen Katheter) wird das getrennte Plasma an dem in Beispiel 1 hergestellten Adsorptions-Mittel vorbeigeführt (wobei die Liganden durch Bindung an die immobilisierten Affinitätspeptide aus dem Plasma abgetrennt werden), bevor es dem Tier wieder zugeführt wird.

### 3. Aβ₄₂-Aptamere

### 3.1 Aktivierung einer Kieselgelsäule mit Tresylchlorid

Die Säule wird mit Aceton durchspült. Zur Aktivierung wird eine wasserfreie Lösung (2 ml Aceton, 1 ml Tresylchlorid, einige Tropfen Pyridin) durch die Säule gegeben (10faches Säulenvolumen) und diese über Nacht auf Eis inkubiert. Anschließend wird die Säule mit dem 20fachen Säulenvolumen 100%Aceton (wasserfrei) durchspült. Die aktivierte Säule kann in 1 mM HCl aufbewahrt werden.

### 3.2 Aktivierung einer Polyethylensäule

Ein Polyethylenschlauch wird mit dem 20fachen Säulenvolumen bei Raumtemperatur mit einer Lösung (2% Kaliumpermanganat (KMnO₄) (w/v) in konzentrierter Schwefelsäure (H₂SO₄)) und anschließend mit destilliertem Wasser durchspült. Zur weiteren Kopplung der Säulenoberfläche können bi- oder polyvalente Moleküle zum Crosslinking verwendet werden, die mindestens eine reaktive Aldehydgruppe (z. B. 1% Glutaraldehyd) aufweisen. Diese werden für 1 h bei 4° C durch die Säule gegeben. Anschließend wird die Reaktion durch reduzierende Bedingungen (z. B. durch Natrium Cyanoborhydrid (0.00025% w/v in 0.15 M NaCl, pH 3,9) stabilisiert.

### 3.3 Koppelung von Aβ₄₂ an die aktivierte Kieselgelsäule

Die Tresylchlorid-aktivierte Säule wird mit 0,1 M Na₂CO₃ (pH 8,5) durchspült. Zur Koppelung wird ein Peptid oder Protein (2 mg/ml 0,1 M Na₂CO₃, pH 8,5) mehrfach für 2 h bei 37° C und anschließend für 4 h auf Eis durch die Säule gegeben. Zur Blockierung freier Bindungstellen der Säule wird anschliessend ein Überschuss von 0,2 M Glycin, pH 8 für durch die Säule gegeben.

### 3.4 Koppelung eines Glykoproteins an die aktivierte Polyethylensäule

Die aktivierte Säule wird mit 0,1 M Na₂CO₃ (pH 8,5) durchspült. Zur Koppelung wird Aβ₄₂ (2 mg/ml 0, 1 M Na₂CO₃, pH 8,5) mehrfach für 2 h bei 37° C und anschließend für 4 h auf Eis durch die Säule gegeben. Zur Blockierung freier Bindungstellen der Säule wird anschließend ein Überschuss von 0,2 M Glycin, pH 8 durch die Säule gegeben. Zur Verbesserung der Reaktion kann 1-Ethyl-3-(3-dimethylaminopropyl)carbodiimid (EDC), 5% w/v zugegeben werden).

### 3.5 Herstellung einer Säule zur Eliminierung unerwünschter Moleküle

Die Tresylchlorid-aktivierte Säule wird mit 0,1 M Na₂CO₃ (pH 8,5) durchspült. Soll gegen ein Molekül mit einer oder mehreren primären oder sekundären Aminen die Elimination erfolgen wird dieses Molekül oder das Gemisch (2 mg/ml 0,1 M Na₂CO₃, pH 8,5) mehrfach über Nacht bei RT durch die Säule gegeben. Zur Blockierung freier Bindungstellen der Säule wird anschließend ein Überschuss von 0,2 M Glycin, pH 8 für durch die Säule gegeben. Wird keine Elimination gegen bestimmte Moleküle mit einer oder mehreren primären oder sekundären Aminen gewünscht, werden alle Bindungstellen der Säule mit Glycin blockiert.

Weitere Derivatisierungsverfahren finden sich beispielsweise in den folgenden Literaturstellen: Patterson, W. J., National Aeronautics and Space Administration, Technical Memorandum, NASA TMX-73311, U.S. Government Printing Office, Washington, D. C., 1976, Ma, S. M., Gregonis, D. E., von Wagenen, R. A., and Andrade, J. D., in "Hydrogels for Medical and Related Applications" (J. D. Andrade, Ed.), Amer. Chem. Soc. Symp. Series, Vol. 31, p. 241, 1976, Harris, J. M., Struck, E. C., Case, M. G., Paley, M. S., Van Alstine, J. M., and Brooks, D. E., J. Polymer Sci., Polymer Chem. Ed., 22, 341 (1984), Regnier and Noel, Regnier, F. E., and Noel, R. J., J. Chromatog. Sci., 14, (1976), Yalpani, M. and Brooks, D. E., J. Polymer Sci., Polymer Chem. Ed., 23, 395 (1985).

### 3.6 Durchführung des Kontaktes der Nukleinsäuren an Aβ₄₂ und Trennung der Säule

Die beschichteten Säulen werden Leckage frei hintereinander geschaltet, erst die Säulen zur Elimination unerwünschter Moleküle, anschließend die Aβ₄₂-Säule. Zur Äquilibrierung wird ein geeigneter Puffer, z. B. eine Pufferlösung (10 mM Tris-HCl, 50 mM KCl, 1,5 mM MgCl₂ und Gelatine 0,001% (w/v), pH 8,3), für 1 h auf Eis über die Säule gegeben. Die Nukleinsäuren der kombinatorischen Nukleinsäurebibliothek werden in 1 ml des gleichen Puffers aufgenommen und zum Aufschmelzen von Doppelsträngen für 10 min auf 95° C erwärmt und anschließend mehrfach (4-30mal) über die Säule gegeben. Anschließend werden die Säulen getrennt und über Nacht auf Eis mit dem gewählten Puffer (s. o.) gewaschen. Die Auftrennung der Säule in Strömungsrichtung erfolgt mit einem geeigneten Schneidwerkzeug.

### 4. Bindungsstudien von Aβ₄₂ an Peptid 1208 (FGFPEHLLVDFLQSLS-C)

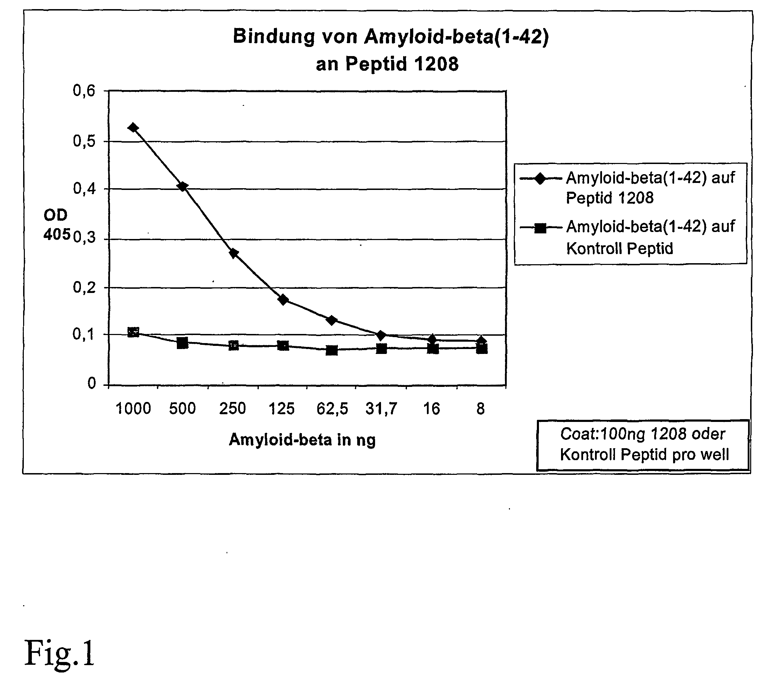

Zur Analyse der Amyloid-beta Bindung an das Peptid 1208 wurde zunächst das Peptid an ein Trägerprotein (BSA) gekoppelt, wobei die Peptidkonzentration 500µMol (ca. 1mg/ml) betrug. Das 1208-BSA Konjugat wurde an eine ELISA Platte gebunden, wobei 100ng Peptid pro Well gebunden wurde. Die ELISA Patte wurde mit PBS, 1% BSA abgesättigt und anschließend wurde die Bindung von Amyloid-beta(1-42) in einem Konzentrationsbereich von 8-1000ng/well analysiert. Gebundenes Amyloid-beta wurde mit einem spezifischen Maus Antikörpers nachgewiesen. Mit Hilfe eines biotinylierten anti-Maus Antikörpers und Streptavidin gekoppelter Peroxidase wurde abschließend die Menge an gebundenem Amyloid-beta quantifiziert. Als Substrat wurde ABTS eingesetzt und in einem ELISA-Reader bei einer Wellenlänge von 405nm analysiert. (Fig. 1)

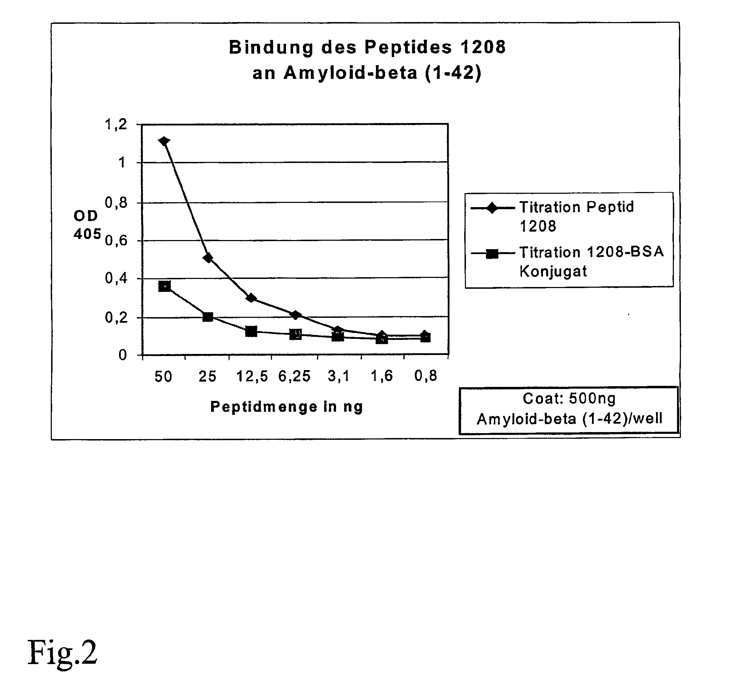

Zur Analyse der Bindung des Peptides 1208, wurde Amyloid-beta (1-42) an eine ELISA Platte gebunden (500ng pro Well). Die ELISA Patte wurde anschließend mit PBS, 1% BSA abgesättigt und dann freies Peptide 1208 bzw. 1208-BSA Konjugat dazugegeben (1,6-50ng). Gebundenes Peptid wurde mit einem spezifischen monoklonalen Antikörper detektiert. Zur abschließenden Quantifizierung wurde ein biotinylierten anti-Maus Antikörper und Streptavidin gekoppelte Peroxidase benutzt. Als Substrat wurde ABTS eingesetzt und in einem ELISA-Reader bei einer Wellenlänge von 405nm analysiert. (Fig. 2)

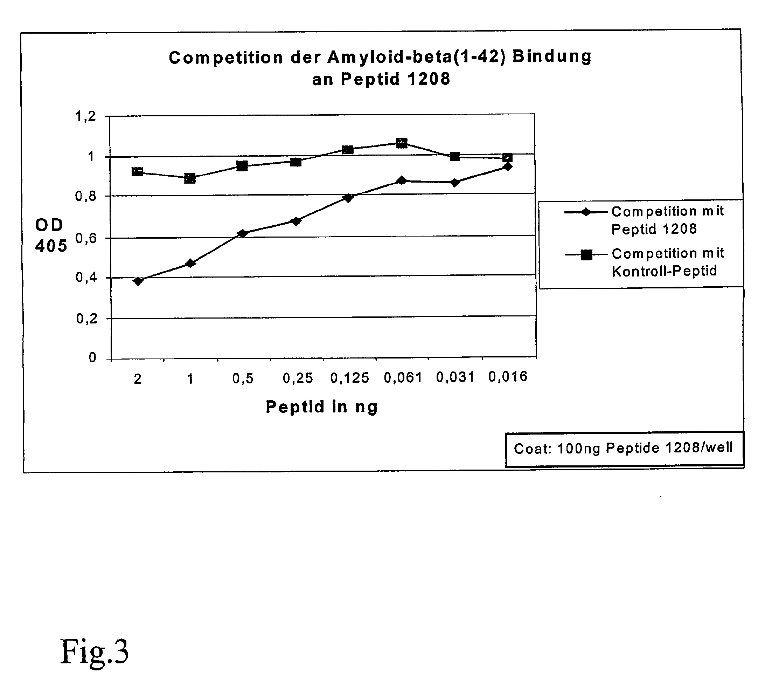

1208-BSA Konjugat wurde an eine ELISA Platte gebunden (100ng Peptid pro Well) und die Patte wurde mit PBS, 1% BSA abgesättigt. Anschließend wurde die Bindung von Amyloid-beta(1-42) (1000ng/well) in Gegenwart von freiem Peptid 1208 oder Kontroll-Peptid (Konzentrationsbereich 30-2000ng/) analysiert. Die Detektion des gebundenen Amyloid-beta erfolgte wie beschrieben. (Fig. 3)

## Patentansprüche

1. Apheresevorrichtung mit einem dem Blut- oder Plasmafluss kontaktierbaren festen Träger, **dadurch gekennzeichnet, dass** der feste Träger einen Amyloid-β-Precursor-Protein (APP) bindenden Rezeptor aufweist.

2. Vorrichtung nach Anspruch 1, **dadurch gekennzeichnet, dass** der APP bindende Rezeptor ausgewählt ist aus anti-APP Antikörpern, anti-Aβ₄₀-Antikörpern, anti-Aβ₄₂-Antikörpern, APP bindenden Proteinen , insbesondere Gelsolin, apoJ oder apoE, APP bindenden Peptiden, APP bindenden Gangliosiden, insbesondere GM1, oder APP bindenden Nukleinsäuren, insbesondere Aptameren, oder Mischungen dieser Rezeptoren.

3. Vorrichtung nach Anspruch 1 oder 2, **dadurch gekennzeichnet, dass** der Träger eine sterile und pyrogenfreie Säule ist.

4. Verwendung einer Vorrichtung nach einem der Ansprüche 1 bis 3, zur Bereitstellung einer Behandlungsvorrichtung zur Behandlung der Alzheimer'schen Erkrankung oder zur Vorbeugung derselben.

## Claims

1. An apheresis device comprising a solid carrier capable of being contacted with the blood or plasma flow, **characterised in that** the solid carrier includes an amyloid-ß-precursor-protein(APP)-binding receptor.

2. The device according to claim 1, **characterised in that** the APP-binding receptor is selected from anti-APP antibodies, anti-Aβ₄₀ antibodies, anti-Aβ₄₂ antibodies, APP-binding proteins, in particular gelsolin, apoJ or apoE, APP-binding peptides, APP-binding gangliosides, in particular GM1, or APP-binding nucleic acids, in particular aptamers, or mixtures of these receptors.

3. The device according to claim 1 or 2, **characterised in that** the carrier is a sterile and pyrogen-free column.

4. Use of a device according to any one of claims 1 to 3, for providing a treatment device for treating Alzheimer's Disease or for preventing the same.

## Revendications

1. Dispositif d'aphérèse avec un support solide pouvant entrer en contact avec un flux de sang ou de plasma, **caractérisé en ce que** le support solide présente un récepteur liant la protéine précurseur de l'amyloïde β (APP).

2. Dispositif selon la revendication 1, **caractérisé en ce que** le récepteur liant l'APP est choisi parmi les anticorps anti-APP, les anticorps anti-Aβ₄₀, les anticorps anti-Aβ₄₂, les protéines liant l'APP, en particulier la gelsoline, apoJ ou apoE, les peptides liant l'APP, les gangliosides liant l'APP, en particulier GM1 ou les acides nucléiques liant l'APP, en particulier les aptamères, ou des mélanges de ces récepteurs.

3. Dispositif selon la revendication 1 ou 2, **caractérisé en ce que** le support est une colonne stérile ou exempte de pyrogène.

4. Utilisation d'un dispositif selon l'une des revendications 1 à 3, pour la préparation d'un dispositif de traitement destiné au traitement de la maladie d'Alzheimer ou à la prévention de cette dernière.
